# FASCICULE DE BREVET EUROPEEN

(11) **EP 0 679 637 B1**
(45) Date de publication et mention de la délivrance du brevet: **10.09.1997**
(21) Numéro de dépôt: 95400594.8
(22) Date de dépôt: 17.03.1995
(51) Int. Cl.: C07C 251/88, C07C 217/40

(54) **Nouveaux dérivés de l'acétaldéhyde, leur procédé de préparation et leur application**
Acetaldehyd-Derivate, Verfahren zu ihrer Herstellung und ihre Verwendung
Acetaldehyde derivatives, process for their preparation and their use

(30) Priorité: 29.04.1994 FR 9405259
(43) Date de publication de la demande: 02.11.1995
(73) Titulaire: SOCIETE FRANCAISE HOECHST, 92800 Puteaux (FR)
(72) Inventeur: Schouteeten, Alain, F-95460 Ezanville (FR); Christidis, Yani, F-75019 Paris (FR)
(74) Mandataire: Rinuy, Santarelli

(56) Documents cités:
- DE-A- 2 233 679
- CHEMICAL ABSTRACTS, vol. 110, no. 9, 1989, Columbus, Ohio, US; abstract no. 74995q, & CAN. J. CHEM., vol.66, no.11, 1988 pages 2839 - 2848 E. LAI ET AL

## Description

La présente invention concerne de nouveaux dérivés de l'acétaldéhyde, leur procédé de préparation et leur application.

La présente invention a pour objet les azines de formule (I). 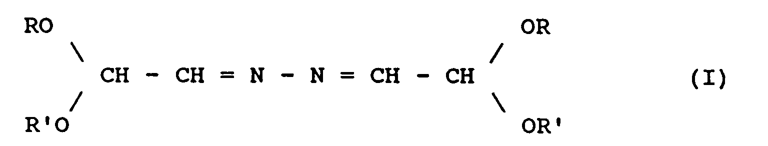 dans laquelle R et R' soit identiques représentent un radical alcoyle contenant de 1 à 4 atomes de carbone ou un radical alcényle contenant de 3 à 5 atomes de carbone, soit forment ensemble un radical de formule (II)

- CHR₁ - (CR₂R₃)ₙ - CHR₄ - (II)

dans laquelle R₁, R₂, R₃ et R₄, identiques ou différents, représentent un atome d'hydrogène ou un radical alcoyle contenant de 1 à 4 atomes de carbone et n représente 0 ou 1, sous leurs différentes formes stéréo-isomères.

Le terme "alcoyle contenant de 1 à 4 atomes de carbone" peut désigner, par exemple, un radical méthyle, éthyle, n-propyle, méthyléthyle, n-butyle, méthyl 1-propyle ou méthyl2-propyle,

Le terme "alcényle contenant de 3 à 5 atomes de carbone" peut désigner, par exemple, un radical allyle, méthylallyle, diméthyl-3,3 allyle.

L'expression "stéréo-isomère" peut désigner par exemple les isomères EE, EZ, ZZ.

L'invention a plus particulièrement pour objet les produits tels que définis ci-dessus, caractérisés en ce que dans la formule (I) R et R', identiques, représentent un radical alcoyle contenant de 1 à 4 atomes de carbone sous leurs différentes formes stéréo-isomères.

Parmi ces derniers produits, l'invention a notamment pour objet :
- la diméthoxy-2,2 acétaldéhyde-azine,
- la diéthoxy-2,2 acétaldéhyde-azine,
- la dibutoxy-2,2 acétaldéhyde-azine,
- la (diméthyl-5,5 oxo-2 dioxanne-1,3)-azine.

Selon l'invention, les produits de formule (I) peuvent être préparés par un procédé caractérisé en ce que l'on fait réagir l'hydrate d'hydrazine avec un acétaldéhyde de formule (III). 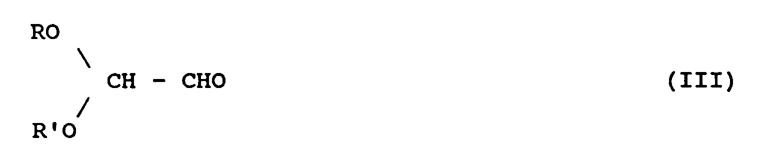 dans laquelle R et R' ont la signification indiquée précédemment pour obtenir un produit de formule (I) correspondant.

Dans des conditions préférentielles de mise en oeuvre de l'invention, le procédé ci-dessus décrit est réalisé :
- à la température ambiante,
- dans de l'eau,
- en utilisant la quantité stoechiométrique d'hydrate d'hydrazine.

Le procédé ci-dessus décrit peut également être mis en oeuvre à une température plus élevée mais généralement inférieure à 100°C et en utilisant un excès d'hydrate d'hydrazine. Il peut être mis en oeuvre également en présence d'un catalyseur basique tel qu'un agent alcalin comme, par exemple, un hydroxyde de métal alcalin, un carbonate de métal alcalin, un hydrogènocarbonate de métal alcalin. Parmi ces agents alcalins, on peut citer plus préférentiellement l'hydroxyde de sodium, le carbonate de sodium, le carbonate de potassium.

Les produits de formule (III) sont des produits soit connus, soit aisément accessibles par des procédés connus comme ceux décrits notamment dans les brevets européens N° 249 530 et 316 672.

Les produits de formule (I) ci-dessus sous leur différentes formes stéréo-isomères présentent d'intéressantes propriétés chimiques ; ils peuvent notamment être transformés aisément par hydrogènolyse en l'amine correspondante de formule (IV). 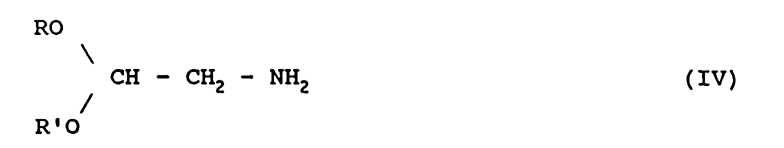 dans laquelle R et R' ont la signification donnée précédemment.

Ces propriétés justifient leur application pour la préparation d'éthylamines substituées de formule (IV) selon notamment un des procédés décrits dans Methoden Der Organischen Chemie - Houben - Weyl - volume XI/1, pages 531-537, Georg Thieme - Stuttgart 1957.

L'invention a également pour objet l'utilisation des azines de formule (I) à l'obtention d'éthylamines substituées de formule (IV). Les éthylamines substituées de formule (IV) sont des matière premières extrêmement intéressantes pour accéder notamment à certains hétérocycles azotés utilisables, par exemple, dans la synthèse de produits doués de propriétés physiologiques tels que certaines phtalazines.

Les exemples suivants illustrent la présente invention sans toutefois la limiter.

### EXEMPLE 1

Dans 50 g (1 mole) d'hydrate d'hydrazine, on introduit sous agitation, en 30 minutes environ et en maintenant la température à 20°C environ par un refroidissement extérieur, 340,3 g d'une solution aqueuse contenant 204,2 g (2 moles) de diméthoxy-2,2-éthanal. L'introduction terminée, on ajoute dans le milieu réactionnel 5,3 g (50 mmoles) de carbonate de sodium anhydre puis on l'abandonne 16 heures sous agitation à la température ambiante. Le produit attendu cristallise spontanément dans le milieu réactionnel, on l'isole par filtration, puis on le lave par empatage avec de l'eau glacée et enfin on le sèche sous pression réduite à poids constant. On obtient ainsi la diméthoxy-2,2 acétaldéhyde-azine sous la forme de cristaux incolores présentant un point de fusion de 56,5 ± 2°C.

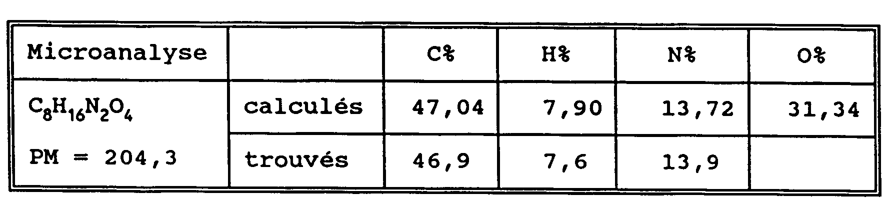

Analyse physique - RMN¹H (CDCl₃)
   3,34 ppm, s, 12 H ; 4,81 ppm, d, J = 4,7 Hz, 2H, 7,53 ppm, d, J = 4,7 Hz, 2H.
spectre en accord avec la structure proposée.

### EXEMPLE 2

On reproduit l'exemple 1 en plaçant le carbonate de sodium dans l'hydrate d'hydrazine avant l'introduction de la solution aqueuse de diméthoxy-2,2 éthanal. On obtient ainsi la diméthoxy-2,2 acétaldéhyde-azine pure avec un excellent rendement.

### EXEMPLE 3

En appliquant le procédé décrit par Ladislas BEREGI, Compt. rend., 224, 1508-1509, (1947) pour la préparation de la propylamine à partir de la propionaldéhyde-azine à la diméthoxy-2,2 acétaldéhyde-azine en solution à 10% en poids dans du méthanol, à 80°C, en présence de 0,1 atome gramme de nickel de Raney, sous une pression d'hydrogène de 10 bars, on obtient avec un rendement sensiblement quantitatif la diméthoxy-2,2 éthylamine pure présentant un point d'ébullition de 136 ± 3°C (littérature J.G ERICKSON et al, J. Amer. Chem. Soc., 77, 6640-6641 (1955), Eb = 139,5 °C).

## Revendications

1. Les azines de formule (I). 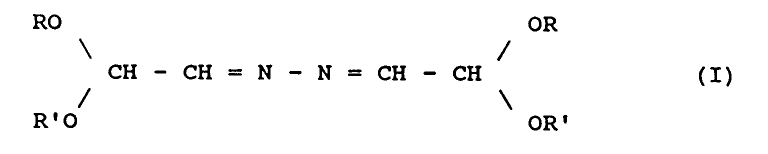 dans laquelle R et R' soit identiques représentent un radical alcoyle contenant de 1 à 4 atomes de carbone ou un radical alcényle contenant de 3 à 5 atomes de carbone, soit forment ensemble un radical de formule (II)
- CHR₁ - ( CR₂R₃ )ₙ - CHR₄ - (II)
dans laquelle R₁, R₂, R₃ et R₄, identiques ou différents, représentent un atome d'hydrogène ou un radical alcoyle contenant de 1 à 4 atomes de carbone et n représente 0 ou 1, sous leurs différentes formes stéréo-isomères.

2. Les azines selon la revendication 1, caractérisées en ce que dans la formule (I), R et R', identiques, représentent un radical alcoyle contenant de 1 à 4 atomes de carbone sous leurs différentes formes stéréo-isomères.

3. La diméthoxy-2,2 acétaldéhyde-azine.

4. La diéthoxy-2,2 acétaldéhyde-azine.

5. La dibutoxy-2,2 acétaldéhyde-azine.

6. La (diméthyl-5,5 oxo-2 dioxanne-1,3)-azine.

7. Procédé de préparation des azines de formule (I) telle que définie à la revendication 1, caractérisé en ce que l'on fait réagir de l'hydrate d'hydrazine sur une acétaldéhyde de formule (III) 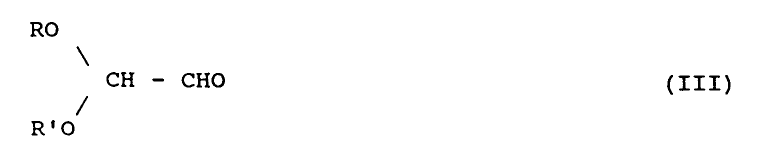 dans laquelle R et R' ont la valeur indiquée à la revendication 1.

8. Procédé selon la revendication 7, caractérisé en ce que l'on fait réagir de l'hydrate d'hydrazine avec une quantité stoechiométrique d'acétaldéhyde de formule (III) dans de l'eau, à la température ambiante.

9. Application des azines de formule (I), telles que définies à la revendication 1, à la préparation d'éthylamines substituées de formule (IV) 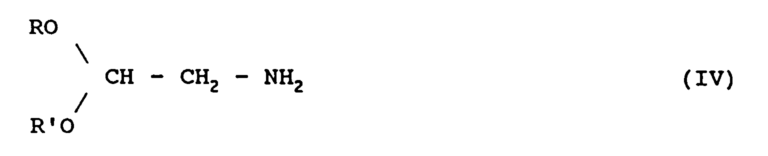 dans laquelle R et R' ont la valeur indiquée à la revendication 1.

10. Application selon la revendication 9 de la diméthoxy-2,2 acétaldéhyde-azine à la préparation de la diméthoxy-2,2 éthylamine.

## Claims

1. The azines of formula (I): 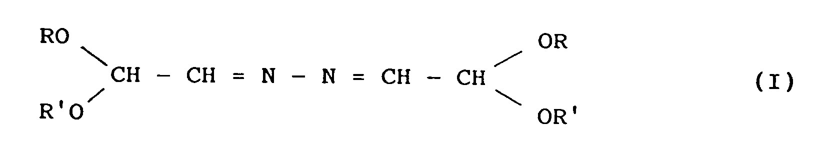 in which R and R' either are identical and represent an alkyl radical containing 1 to 4 carbon atoms or an alkenyl radical containing 3 to 5 carbon atoms, or together form a radical of formula (II):
- CHR₁ - (CR₂R₃)ₙ - CHR₄ - (II)
in which R₁, R₂, R₃ and R₄, identical or different, represent a hydrogen atom or an alkyl radical containing 1 to 4 carbon atoms and n represents 0 or 1, in all their different stereoisomer forms.

2. The azines according to claim 1, characterized in that in formula (I), R and R', being identical, represent an alkyl radical containing 1 to 4 carbon atoms in their different stereoisomer forms.

3. 2,2-dimethoxy acetaldehyde-azine.

4. 2,2-diethoxy acetaldehyde-azine.

5. 2,2-dibutoxy acetaldehyde-azine.

6. (5,5-dimethyl 2-oxo 1,3-dioxane)-azine.

7. Preparation process for azines of formula (I) as defined in claim 1, characterized in that hydrazine hydrate is reacted on an acetaldehyde of formula (III): 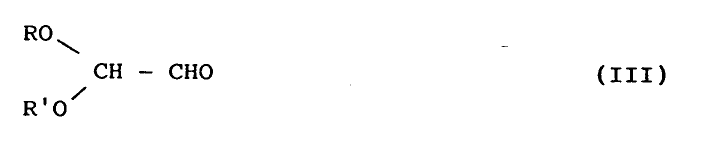 in which R and R' have the value indicated in claim 1.

8. Process according to claim 7, characterized in that hydrazine hydrate is reacted with a stoichiometric quantity of the acetaldehyde of formula (III) in water, at ambient temperature.

9. Use of the azines of formula (I), as defined in claim 1, for the preparation of substituted ethylamines of formula (IV): 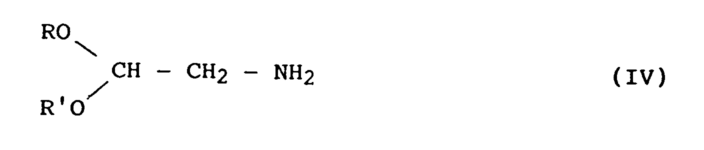 in which R and R' have the value indicated in claim 1.

10. Use according to claim 9 of 2,2-dimethoxy acetaldehyde-azine for the preparation of 2,2-dimethoxy ethylamine.

## Patentansprüche

1. Azine der Strukturformel (I): 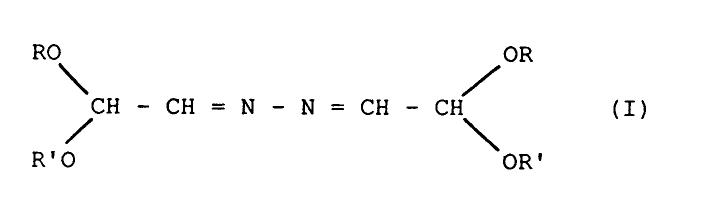 in ihren verschiedenen stereoisomeren Formen,
worin R und R' entweder gleich sind und für einen Alkylrest mit 1 bis 4 Kohlenstoffatomen oder einen Alkenylrest mit 3 bis 5 Kohlenstoffatomen stehen, oder gemeinsam einen Rest der Strukturformel (II)
- CHR₁ - (CR₂R₃)ₙ - CHR₄ - (II)
bilden, worin R₁, R₂, R₃ und R₄ gleich oder verschieden sind und für ein Wasserstoffatom oder einen Alkylrest mit 1 bis 4 Kohlenstoffatomen stehen, n für 0 oder 1 steht.

2. Azine nach Anspruch 1 in ihren verschiedenen stereoisomeren Formen, dadurch gekennzeichnet, daß in der Strukturformel (I) R und R' gleich sind und für einen Alkylrest mit 1 bis 4 Kohlenstoffatomen stehen.

3. 2,2-Dimethoxyacetaldehyd-azin.

4. 2,2-Diethoxyacetaldehyd-azin.

5. 2,2-Dibutoxyacetaldehyd-azin.

6. (5,5-Dimethyl-2-oxo-1,3-dioxan)-azin.

7. Verfahren zur Herstellung von Azinen der in Anspruch 1 angegebenen Strukturformel (I), dadurch gekennzeichnet, daß man Hydrazinhydrat mit einem Acetaldehyd der Strukturformel (III) 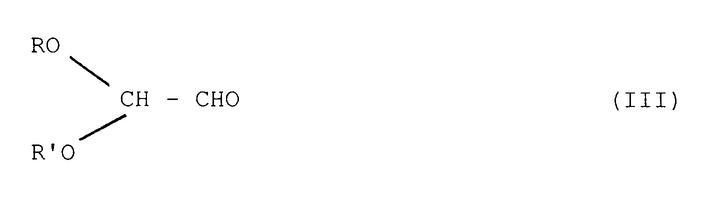 umsetzt, worin R und R' die in Anspruch 1 angegebene Bedeutung haben.

8. Verfahren nach Anspruch 7, dadurch gekennzeichnet, daß man das Hydrazinhydrat mit einer stöchiometrischen Menge des Acetaldehyds der Strukturformel (III) in Wasser und bei Umgebungstemperatur umsetzt.

9. Verwendung der Azine der in Anspruch 1 angegebenen Strukturformel (I) zur Herstellung von substituierten Ethylaminen der Strukturformel (IV) 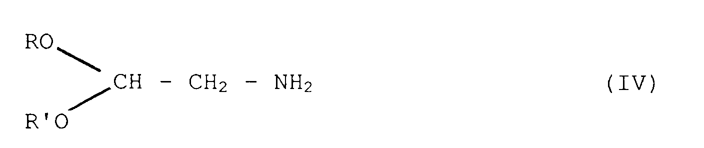 worin R und R' die in Anspruch 1 angegebene Bedeutung haben.

10. Verwendung nach Anspruch 9 von 2,2-Dimethoxyacetaldehydazin zur Herstellung von 2,2-Dimethoxyethylamin.
